# EUROPEAN PATENT SPECIFICATION

(11) **EP 3 348 541 B1**
(45) Date of publication and mention of the grant of the patent: **30.09.2020**
(21) Application number: 16844166.5
(22) Date of filing: 23.08.2016
(51) Int. Cl.: C07C 43/295, C08K 5/13, C08L 23/00, C08L 101/00, C09K 15/08

(54) **STABILIZER FOR ORGANIC MATERIALS**
STABILISATOR FÜR ORGANISCHE MATERIALIEN
STABILISATEUR POUR MATIÈRES ORGANIQUES

(30) Priority: 07.09.2015 JP 2015175839
(43) Date of publication of application: 18.07.2018
(73) Proprietor: Sumitomo Chemical Company, Limited, Tokyo 104-8260 (JP)
(72) Inventor: OZTURK, Orhan, Tokyo 104-8260 (JP); KIMURA, Natsuko, Osaka-shi Osaka 554-8558 (JP); MATSUMOTO, Shuhei, Ehime 792-0015 (JP)
(74) Representative: Vossius & Partner Patentanwälte Rechtsanwälte mbB
(86) International application number: PCT/JP2016/074437
(87) International publication number: WO 2017/043303

(56) References cited:
- GB-A- 1 342 455
- JP-A- H01 168 643
- JP-A- 2001 081 250
- MASASHI TASHIRO ET AL: "Selective preparation. 31. Oxidative coupling of 2-halo-4,6-di-tert-butylphenols with potassium hexacyanoferrate(III) in benzene", JOURNAL OF ORGANIC CHEMISTRY, vol. 46, no. 19, 1 September 1981 (1981-09-01), pages 3784-3789, XP055556558, ISSN: 0022-3263, DOI: 10.1021/jo00332a004
- DAVID R. ARMSTRONG ET AL: "Oxidative coupling of phenols. Part 10. The role of steric effects in the formation of C-O coupled products", JOURNAL OF THE CHEMICAL SOCIETY, PERKIN TRANSACTIONS 2: PHYSICAL ORGANIC CHEMISTRY, no. 5, 1 January 1983 (1983-01-01), pages 587-589, XP055556622, GB ISSN: 0300-9580, DOI: 10.1039/P29830000587
- MASASHI TASHIRO ET AL.: 'Selective Preparation. 31. Oxidative Coupling of 2-Halo-4,6-di-tert-butylphenols with Potassium Hexacyanoferrate(II1) in Benzene 1,2' JOURNAL OF ORGANIC CHEMISTRY vol. 46, no. 19, 1981, pages 3784 - 3789, XP055369137

## Description

### [Technical Field]

The present invention relates to a compound useful as a stabilizer for an organic material.

### [Background Art]

Organic materials such as thermoplastic resins and the like are deteriorated by the action of heat and oxygen during processing into products and when in use after processing. To improve stability during processing (processing stability) and stability in use (e.g., oxidation resistance), therefore, a stabilizer is added to the organic materials. As such stabilizer, various phenolic antioxidants (e.g., "IRGANOX (registered trade mark) 1076" manufactured by BASF) is known (e.g., patent document 1).

GB 1 342 455 A discloses the preparation of phenoxyphenols which may be used as antioxidants.

### [Document List]

### [Patent document]

patent document 1: JP-A-2001-81250

### [SUMMARY OF THE INVENTION]

### [Problems to be Solved by the Invention]

The present invention aims to provide a compound capable of improving processing stability and oxidation resistance of organic material compositions, and useful as a stabilizer.

### [Means of Solving the Problems]

The present inventors have conducted intensive studies and found that a compound represented by the following formula (I) (hereinafter sometimes to be abbreviated as "compound (I)") can improve processing stability and oxidation resistance of an organic material composition, and is useful as a stabilizer. The present invention predicated on this finding is as described below.
[1] A compound represented by the formula (I): 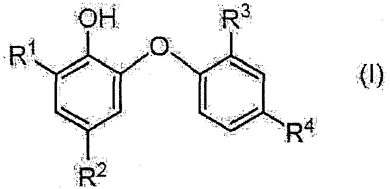 in the formula (I), R¹ - R⁴ are each independently a t-butyl group or a t-pentyl group.
[2] The compound of the aforementioned [1], wherein R¹ - R⁴ are each a t-butyl group.
[3] A stabilizer for an organic material, comprising the compound represented by the formula (I) of the aforementioned [1] or [2].
[4] The stabilizer of the aforementioned [3], wherein the organic material is a thermoplastic resin.
[5] The stabilizer of the aforementioned [4], wherein the thermoplastic resin is a polyolefin, a polyamide, a polyester, or a polycarbonate.
[6] The stabilizer of the aforementioned [4], wherein the thermoplastic resin is a polyolefin.
[7] The stabilizer of the aforementioned [5] or [6], wherein the polyolefin is polyethylene or polypropylene.
[8] A method of stabilizing an organic material, comprising mixing the organic material and the compound represented by the formula (I) of the aforementioned [1] or [2].
[9] The method of the aforementioned [8], wherein the organic material is a thermoplastic resin.
[10] The method of the aforementioned [9], wherein the thermoplastic resin is a polyolefin, a polyamide, a polyester, or a polycarbonate.
[11] The method of the aforementioned [9], wherein the thermoplastic resin is a polyolefin.
[12] The method of the aforementioned [10] or [11], wherein the polyolefin is polyethylene or polypropylene.
[13] The method of any one of the aforementioned [8] - [12], wherein an amount of the compound represented by the formula (I) is 0.01 - 5 parts by weight per 100 parts by weight of the organic material.
[14] The method of any one of the aforementioned [8] - [12], wherein an amount of the compound represented by the formula (I) is 0.03 - 3 parts by weight per 100 parts by weight of the organic material.
[15] The method of any one of the aforementioned [8] - [12], wherein an amount of the compound represented by the formula (I) is 0.05 - 1 part by weight per 100 parts by weight of the organic material.
[16] A stabilized organic material composition comprising an organic material and the compound represented by the formula (I) of the aforementioned [1] or [2].
[17] The organic material composition of the aforementioned [16], wherein the organic material is a thermoplastic resin.
[18] The organic material composition of the aforementioned [17], wherein the thermoplastic resin is a polyolefin or, a polyamide, a polyester, or a polycarbonate.
[19] The organic material composition of the aforementioned [17], wherein the thermoplastic resin is a polyolefin.
[20] The organic material composition of the aforementioned [18] or [19], wherein the polyolefin is polyethylene or polypropylene.
[21] The composition of any one of the aforementioned [16] - [20], wherein an amount of the compound represented by the formula (I) is 0.01 - 5 parts by weight per 100 parts by weight of the organic material.
[22] The composition of any one of the aforementioned [16] - [20], wherein an amount of the compound represented by the formula (I) is 0.03 - 3 parts by weight per 100 parts by weight of the organic material.
[23] The composition of any one of the aforementioned [16] - [20], wherein an amount of the compound represented by the formula (I) is 0.05 - 1 part by weight per 100 parts by weight of the organic material.

### [Effect of the Invention]

The compound (I) of the present invention can improve processing stability and oxidation resistance of an organic material composition.

### [Description of Embodiments]

### <Compound (I)>

The present invention provides a compound represented by the formula (I).

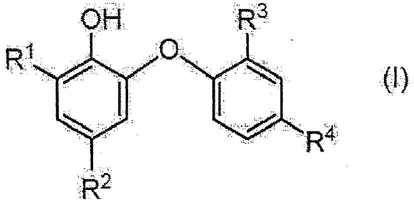

In the formula (I), R¹ - R⁴ are each independently a t-butyl group or a t-pentyl group. It is preferable that all of R¹ - R⁴ be the same group (t-butyl group or t-pentyl group), more preferably, R¹ - R⁴ are t-butyl groups.

Compound (I) wherein R¹ - R⁴ are t-butyl groups (i.e., 2,4-di-t-butyl-6-(2,4-di-t-butylphenoxy)phenol) can be produced by the method described in the below-mentioned Examples. Other compounds (I) can be produced in the same manner as in the method described in the below-mentioned Examples except that different starting materials are used.

### <stabilizer for organic material, stabilizing method of organic material and stabilized organic material composition>

The compound (I) of the present invention can suppress thermal degradation and oxidation degradation of an organic material, and can improve processing stability and oxidation resistance of an organic material composition. Therefore, the present invention provides (1) a stabilizer for an organic material, which contains compound (I), (2) a method of stabilizing an organic material, comprising mixing an organic material and compound (I), as well as (3) a stabilized organic material composition comprising an organic material and compound (I). In any of the stabilizer, stabilizing method and organic material composition of the present invention, only one kind of compound (I) may be used, or two or more kinds thereof may be used in combination.

Only one kind of an organic material may be used, or two or more kinds thereof may be used in combination. Examples of the organic material include those indicated below, though the present invention is not limited by the following organic materials.
thermoplastic resins such as
(1) polyethylene, for example, high-density polyethylene (HD-. PE), low-density polyethylene (LD-PE), linear low-density polyethylene (LLDPE)
(2) polypropylene
(3) methylpentene polymer
(4) EEA (ethylene/ethyl acrylate copolymer) resin
(5) ethylene/vinyl acetate copolymer resin
(6) polystyrenes, for example, polystyrene, poly(p-methylstyrene), poly(α-methylstyrene)
(7) AS (acrylonitrile/styrene copolymer) resin
(8) ABS (acrylonitrile/butadiene/styrene copolymer) resin
(9) AAS (special acrylic rubber/acrylonitrile/styrene copolymer) resin
(10) ACS (acrylonitrile/chlorinated polyethylene/styrene copolymer) resin
(11) chlorinated polyethylene, polychloroprene, chlorinated rubber
(12) polyvinyl chloride, polyvinyldene chloride
(13) methacrylic resin
(14) ethylene/vinyl alcohol copolymer resin
(15) fluororesin
(16) polyacetal
(17) grafted polyphenylene ether resin and polyphenylene sulfide resin
(18) polyurethane
(19) polyamide
(20) polyester, for example, poly(ethylene terephthalate), poly(butylene terephthalate)
(21) polycarbonate
(22) polyacrylate
(23) polysulfone, polyether ether ketone, polyether sulfone
(24) aromatic polyester resin
thermosetting resins such as
(25) epoxy resin
(26) diallyl phthalate prepolymer
(27) silicone resin
(28) unsaturated polyester resin
(29) acrylic modified benzoguanamine resin
(30) benzoguanamine/melamine resin
(31) urea resin
(32) polybutadiene
(33) 1,2-polybutadiene
(34) polyisoprene
(36) butadiene/acrylonitrile copolymer
(37) ethylene/propylene copolymer
(38) silicone rubber
(39) epichlorohydrin rubber
(40) acrylic rubber
(41) natural rubber
(42) chlororubber paint
(43) polyester resin paint
(44) urethane resin paint
(45) epoxy resin paint
(46) acrylic resin paint
(47) vinyl resin paint
(48) aminoalkyd resin paint
(49) alkyd resin paint
(50) nitrocellulose resin paint
(51) oil paint
(52) wax
(53) lubricating oil

As the organic material, a thermoplastic resin is preferable, polyolefin (e.g., polyethylene, polypropylene) and polyamide, polyester, or polycarbonate are more preferable, polyolefin is further preferable, and polyethylene and polypropylene are particularly preferable.

The melt flow rate (MFR) at temperature 190°C and load 21.18N of polyethylene is preferably 0.01 - 40 g/10 min, more preferably 0.1 - 20 g/10 min, further preferably 0.5 - 5 g/10 min, from the aspects of processability. The above-mentioned MFR can be measured according to JIS K6922-2.

The melt flow rate (MFR) at temperature 230°C and load 21.18N of polypropylene is preferably 0.01 - 40 g/10 min, more preferably 0.1 - 20 g/10 min, further preferably 10.0 - 18.0 g/10 min, from the aspects of processability. The above-mentioned MFR can be measured according to JIS K6922-2.

Examples of the polyolefin include one obtained by radical polymerization, and one produced by polymerization using a catalyst containing a metal of group IVb, Vb, VIb or VIII in the periodic table. Examples of the aforementioned catalyst include a metal complex having one or more ligands (e.g., oxide, halogen compound, alcoholate, ester, aryl, each of which is coordinated by π or σ bond). The aforementioned metal complex may be used as it is, or may be carried on a carrier such as magnesium chloride, titanium chloride, alumina, silicon oxide and the like. As the catalyst, Ziegler-Natta catalyst, TNZ catalyst, metallocene catalyst, and Phillips catalyst are preferable.

Polyamide as an engineering resin may be any as long as it has an amide bond in the polymer chain, and melted by heating. As the polyamide, one produced by any method such as condensation reaction of diamines and dicarboxylic acids, condensation reaction of aminocarboxylic acids, ring opening polymerization of lactams, and the like. Representative examples of polyamide include nylon 66, nylon 69, nylon 610, nylon 612, poly-bis(p-aminocyclohexyl)methanedodecamide, nylon 46, nylon 6, nylon 12, nylon 66/6, and nylon 6/12.

Polyester as an engineering resin may be any as long as it has an ester bond in the polymer chain, and melted by heating. Examples of the polyester include those obtained by polycondensation of dicarboxylic acids and dihydroxy compounds, and the like. Polyester may be any of a homopolymer and a copolymer. Representative examples of polyester include poly(ethylene terephthalate) and poly(butylene terephthalate).

Polycarbonate as an engineering resin may be any as long as it has a carbonate bond in the polymer chain, and melted by heating. Examples of the polycarbonate include those obtained by reacting an aromatic hydroxy compound, or an aromatic hydroxy compound and a small amount of a polyhydroxy compound with a carbonate precursor such as phosgene and diphenyl carbonate in the presence of a solvent, an acid acceptor and a molecular weight modifier. Polycarbonate may be any of a homopolymer and a copolymer, and may be any of a linear and a branched chain.

The amount of compound (I) in the organic material composition of the present invention is preferably 0.01 - 5 parts by weight, more preferably 0.03 - 3 parts by weight, further preferably 0.05 - 1 part by weight, per 100 parts by weight of the organic material. When the amount is less than 0.01 part by weight, stabilization of an organic material is not necessarily sufficient and, when it exceeds 5 parts by weight, improvement of stabilization comparable thereto cannot be achieved, and is economically disadvantageous. In the stabilizer and stabilizing method of the present invention, it is also desirable to use compound (I) in the aforementioned preferable amounts.

The stabilizer and organic material composition of the present invention may contain an additive other than compound (I) (hereinafter sometimes to be abbreviated as "other additive") as long as the effect of the invention (improvement of processing stability and oxidation resistance) is not inhibited. Only one kind of other additive may be used or two or more kinds thereof may be used in combination. The amount of other additive in the stabilizer of the present invention is, for example, 0 - 90 wt%, preferably 0.01 - 30 wt%. The amount of other additive in the organic material composition of the present invention is, for example, 0 - 4.5 parts by weight, preferably 0.01 - 2.5 parts by weight, per 100 parts by weight of the organic material.

Examples of other additive include amines, acid-binding metal salt, phenolic antioxidant, sulfur antioxidant, phosphorus antioxidant, ultraviolet absorber, light stabilizer, metal deactivator, peroxide scavenger, polyamide stabilizer, hydroxyamine, neutralizing agent, lubricant, nucleating agent, filler, plasticizer, flame-retardant, antistatic agent, antiblocking agent, surfactant, processing aid, foaming agent, emulsifier, gloss agent, coloring-improving agent (e.g., 9,10-dihydro-9-oxa-10-phosphophenanthrene-10-oxide), and benzofurans and indolines described in US-B-4,325,853, 4,338,244, 5,175,312, 5,216,053, 5,252,643, 4,316,611, DE-A-4, 316, 622, 4,316,876, EP-A-589,839, 591,102. Only one kind of these may be used or two or more kinds thereof may be used in combination.

Examples of the amines include the following:
trialkanolamines such as triethanolamine, tripropanolamine, triisopropanolamine and the like, dialkanolamines such as diethanolamine, dipropanolamine, diisopropanolamine, tetraethanolethylenediamine, tetraisopropanolethylenediamine and the like, monoalkanolamines such as dibutylethanolamine, dibutylisopropanolamine and the like, aromatic amines such as 1,3,5-trimethyl-2,4,6-triazine and the like, alkylamines such as dibutylamine, piperidine, 2,2,6,6-tetramethylpiperidine, 4-hydroxy-2,2,6,6-tetramethylpiperidine and the like, polyalkylenepolyamines such as hexamethylenetetramine, triethylenediamine, triethylenetetramine, tetraethylenepentamine and the like, the below-mentioned hindered amine light stabilizer.

As amines, long chain aliphatic amine described in JP-A-61-63686, a compound containing the steric hindered amino group described in JP-A-6-329830, a hindered piperidinyl light stabilizer described in JP-A-7-90270, organic amine described in JP-A-7-278164 can be used.

Examples of the acid-binding metal salt include hydrotalcites. Examples of the hydrotalcites include those represented by the following formula:
M²⁺₁₋ₓ•M³⁺ₓ•(OH⁻)₂• (Aⁿ⁻)_{x/n}•pH₂O
wherein M²⁺ is Mg, Ca, Sr, Ba, Zn, Pb, Sn and/or Ni, M³⁺ is Al, B or Bi, n is a value of 1 - 4, x is a value of 0 - 0.5, p is a value of 0 - 2, and Aⁿ⁻ is anion of n-valence.

Examples of Aⁿ⁻ include OH⁻, Cl⁻, Br⁻, I⁻, ClO₄⁻, HCO₃⁻, C₆H₅COO⁻, CO₃²⁻, SO²⁻, ⁻OOCCOO⁻, (CHOHCOO)₂²⁻, C₂H₄(COO)₂²⁻, (CH₂COO)₂²⁻, CH₃CHOHCOO⁻, SiO₃²⁻, SiO₄⁴⁻, Fe(CN)₆⁴⁻, BO³⁻, PO₃³⁻ and HPO₄²⁻.

Of hydrotalcites, one represented by the following formula:
Mg₁₋ₓAlₓ(OH)₂(CO₃)_{x/2}•pH₂O
wherein x and p are as defined above, is preferable.

Hydrotalcites may be natural products or synthetic products, and can be used irrespective of the crystal structures thereof, crystal particle size thereof and the like.

Furthermore, ultrafine zinc oxide described in JP-A-6-329830, inorganic compounds described in JP-A-7-278164 can also be used as an acid-binding metal salt.

Examples of the phenolic antioxidant include the following:

### (1) alkylated monophenol

2,6-di-t-butyl-4-methylphenol, 2,4,6-tri-t-butylphenol, 2,6-di-t-butylphenol, 2-t-butyl-4,6-dimethylphenol, 2,6-di-t-butyl-4-ethylphenol, 2,6-di-t-butyl-4-n-butylphenol, 2,6-di-t-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-di-t-butyl-4-methoxymethylphenol, 2,6-di-nonyl-4-methylphenol, 2,4-dimethyl-6-(1'-methylundecyl-1'-yl)phenol, 2,4-dimethyl-6-(1'-methylheptadecyl-1'-yl)phenol, 2,4-dimethyl-6-(1'-methyltridecyl-1'-yl)phenol and mixtures thereof.

### (2) alkylthiomethylphenol

2,4-dioctylthiomethyl-6-t-butylphenol, 2,4-dioctylthiomethyl-6-methylphenol, 2,4-dioctylthiomethyl-6-ethylphenol, 2,6-didodecylthiomethyl-4-nonylphenol and mixtures thereof.

### (3) hydroquinone and alkylated hydroquinone

2,6-di-t-butyl-4-methoxyphenol, 2,5-di-t-butylhydroquinone, 2,5-di-t-amylhydroquinone, 2,6-diphenyl-4-octadecyloxyphenol, 2,6-di-t-butylhydroquinone, 2,5-di-t-butyl-4-hydroxyanisole, 3,5-di-t-butyl-4-hydroxyphenyl stearate, bis(3,5-di-t-butyl-4-hydroxyphenyl)adipate and mixtures thereof.

### (4) tocopherol

α-tocopherol, β-tocopherol, γ-tocopherol, δ-tocopherol and mixtures thereof.

### (5) hydroxylated thiodiphenylether

2,2'-thiobis(6-t-butylphenol), 2,2'-thiobis(4-methyl-6-t-butylphenol), 2,2'-thiobis(4-octylphenol), 4,4'-thiobis(3-methyl-6-t-butylphenol), 4,4'-thiobis(2-methyl-6-t-butylphenol), 4,4'-thiobis(3,6-di-t-amylphenol), 4,4'-(2,6-dimethyl-4-hydroxyphenyl) disulfide.

### (6) alkylidene bisphenol and derivative thereof

2,2'-methylene bis(4-methyl-6-t-butylphenol), 2,2'-methylene bis(4-ethyl-6-t-butylphenol), 2,2'-methylene bis[4-methyl-6-(α-methylcyclohexyl)phenol)], 2,2'-methylene bis(4-methyl-6-cyclohexylphenol), 2,2'-methylene bis(4-methyl-6-nonylphenol), 2,2'-methylene bis(4,6-di-t-butylphenol), 2,2'-ethylidene bis(4,6-di-t-butylphenol), 2,2'-ethylidene bis(4-isobutyl-6-t-butylphenol), 2,2'-methylene bis[6-(α-methylbenzyl)-4-nonylphenol], 2,2'-methylene bis[6-(α,α-dimethylbenzyl)-4-nonylphenol], 4,4'-methylene bis(6-t-butyl-2-methylphenol), 4,4'-methylene bis(2,6-di-t-butylphenol), 4,4'-butylidene bis(3-methyl-6-t-butylphenol), 1,1-bis(4-hydroxyphenyl)cyclohexane, 1,1-bis(5-t-butyl-4-hydroxy-2-methylphenyl)butane, 2,6-bis(3-t-butyl-5-methyl-2-hydroxybenzyl)-4-methylphenol, 1,1,3-tris(5-t-butyl-4-hydroxy-2-methylphenyl)butane, 1,1-bis(5-t-butyl-4-hydroxy-2-methylphenyl)-3-n-dodecylmercaptobutane, ethylene glycol bis[3,3-bis-3'-t-butyl-4'-hydroxyphenyl)butyrate], bis(3-t-butyl-4-hydroxy-5-methylphenyl)dicyclopentadiene, bis[2-(3'-t-butyl-2'-hydroxy-5'-methylbenzyl)-6-t-butyl-4-methylphenyl]terephthalate, 1,1-bis(3,5-dimethyl-2-hydroxyphenyl)butane, 2,2-bis(3,5-di-t-butyl-4-hydroxyphenyl)propane, 2,2-bis(5-t-butyl-4-hydroxy-2-methylphenyl)-4-n-dodecylmercaptobutane, 1,1,5,5-tetra(5-t-butyl-4-hydroxy-2-methylphenyl)pentane, 2-t-butyl-6-(3'-t-butyl-5'-methyl-2'-hydroxybenzyl)-4-methylphenyl acrylate, 2,4-di-t-pentyl-6-[1-(2-hydroxy-3,5-di-t-pentylphenyl)ethy1]phenyl acrylate and mixtures thereof.

### (7) 0-, N- and S-benzyl derivatives

3,5,3',5'-tetra-t-butyl-4,4'-dihydroxydibenzyl ether, octadecyl-4-hydroxy-3,5-dimethyibenzylmercaptoacetate, tris(3,5-di-t-butyl-4-hydroxybenzyl)amine, bis(4-t-butyl-3-hydroxy-2,6-dimethylbenzyl)dithioterephthalate, bis(3,5-di-t-butyl-4-hydroxybenzyl)sulfide, isooctyl-3,5-di-t-butyl-4-hydroxybenzylmercaptoacetate and mixtures thereof.

### (8) hydroxybenzylated malonate derivative

dioctadecyl-2,2-bis(3,5-di-t-butyl-2-hydroxybenzyl)malonate, dioctadecyl-2-(3-t-butyl-4-hydroxy-5-methylbenzyl)malonate, didodecylmercaptoethyl-2,2-bis(3,5-di-t-butyl-4-hydroxybenzyl)malonate, bis[4-(1,1,3,3-tetramethylbutyl)phenyl]-2,2-bis(3,5-di-t-butyl-4-hydroxybenzyl)malonate and mixtures thereof.

### (9) aromatic hydroxybenzyl derivative

1,3,5-trimethyl-2,4,6-tris(3,5-di-t-butyl-4-hydroxybenzyl)benzene, 1,4-bis(3,5-di-t-butyl-4-hydroxybenzyl)-2,3,5,6-tetramethylbenzene, 2,4,6-tris(3,5-t-butyl-4-hydroxybenzyl)phenol and mixtures thereof.

### (10) triazine derivative

2,4-bis(n-octylthio)-6-(4-hydroxy-3,5-di-t-butylanilino)-1,3,5-triazine, 2-n-octylthio-4,6-bis(4-hydroxy-3,5-di-t-butylanilino)-1,3,5-triazine, 2-n-octylthio-4,6-bis(4-hydroxy-3,5-di-t-butylphenoxy)-1,3,5-triazine, 2,4,6-tris(3,5-di-t-butyl-4-phenoxy)-1,3,5-triazine, tris(4-t-butyl-3-hydroxy-2,6-dimethylbenzyl)isocyanurate, tris(3,5-di-t-butyl-4-hydroxybenzyl)isocyanurate, 2,4,6-tris(3,5-di-t-butyl-4-hydroxyphenylethyl)-1,3,5-triazine, 2,4,6-tris(3,5-di-t-butyl-4-hydroxyphenylpropyl)-1,3,5-triazine, tris(3,5-dicyclohexyl-4-hydroxybenzyl)isocyanurate, tris[2-(3',5'-di-t-butyl-4'-hydroxycinnamoyloxy)ethyl]isocyanurate and mixtures thereof.

### (11) benzylphosphonate derivative

dimethyl-3,5-di-t-butyl-4-hydroxybenzylphosphonate, diethyl-3,5-di-t-butyl-4-hydroxybenzylphosphonate, dioctadecyl-3,5-di-t-butyl-4-hydroxybenzylphosphonate, dioctadecyl-5-t-butyl-4-hydroxy-3-methylbenzylphosphonate, calcium salt of 3,5-di-t-butyl-4-hydroxybenzylphosphonic acid monoester and mixtures thereof.

### (12) acylaminophenol derivative

4-hydroxylauryl anilide, 4-hydroxystearic anilide, octyl-N-(3,5-di-t-butyl-4-hydroxyphenyl)carbamate and mixtures thereof.

### (13) ester of β-(3,5-di-t-butyl-4-hydroxyphenyl)propionic acid and the following monovalent or polyvalent alcohol

methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol, spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2,2,2]octane and mixtures thereof.

### (14) ester of β-(5-t-butyl-4-hydroxy-3-methylphenyl)propionic acid and the following monovalent or polyvalent alcohol

methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol, spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2,2,2]octane and mixtures thereof.

### (15) ester of β-(3,5-dicyclohexyl-4-hydroxyphenyl)propionic acid and the following monovalent or polyvalent alcohol

methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol, spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2,2,2]octane and mixtures thereof.

### (16) ester of 3,5-di-t-butyl-4-hydroxyphenylacetic acid and the following monovalent or polyvalent alcohol

methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol, spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2,2,2]octane and mixtures thereof.

### (17) β-(3,5-di-t-butyl-4-hydroxyphenyl)propionic acid amide

N,N'-bis[3-(3',5'-di-t-butyl-4'-hydroxyphenyl)propionyl]hydrazine, N,N'-bis[3-(3',5'-di-t-butyl-4'-hydroxyphenyl)propionyl]hexamethylenediamine, N,N' - bis[3-(3',5'-di-t-butyl-4'-hydroxyphenyl)propionyl]trimethylenediamine and mixtures thereof.

Examples of the sulfur antioxidant include the following:
dilauryl 3,3'-thiodipropionate, tridecyl 3,3'-thiodipropionate, dimyristyl 3,3'-thiodipropionate, distearyl 3,3'-thiodipropionate, lauryl stearyl 3,3'-thiodipropionate, neopentanetetrayl tetrakis(3-laurylthiopropionate).

Examples of the phosphorus antioxidant include the following:
triphenyl phosphite, tris(nonylphenyl) phosphite, tris(2,4-di-t-butylphenyl) phosphite, trilauryl phosphite, trioctadecyl phosphite, distearyl pentaerythritol diphosphite, diisodecyl pentaerythritol diphosphite, bis(2,4-di-t-butylphenyl) pentaerythritol diphosphite, bis(2,4-di-t-butyl-6-methylphenyl) pentaerythritol diphosphite, bis(2,6-di-t-butyl-4-methylphenyl) pentaerythritol diphosphite, bis(2,4,6-tri-t-butylphenyl) pentaerythritol diphosphite, tristearyl sorbitol triphosphite, tetrakis(2,4-di-t-butylphenyl)-4,4'-diphenylene diphosphonite, 2,2'-methylene bis(4,6-di-t-butylphenyl) 2-ethylhexyl phosphite, 2,2'-ethylidene bis(4,6-di-t-butylphenyl) fluoro phosphite, bis(2,4-di-t-butyl-6-methylphenyl) ethyl phosphite, bis(2,4-di-t-butyl-6-methylphenyl) methyl phosphite, 2-(2,4,6-tri-t-butylphenyl)-5-ethyl-5-butyl-1,3,2-oxaphosphorinan, 2,2',2"-nitrilo[triethyl-tris(3,3',5,5'-tetra-t-butyl-1,1'-biphenyl-2,2'-diyl) phosphite, 2,4,8,10-tetra-t-butyl-6-[3-(3-methyl⁻4-hydroxy-5-t-butylphenyl)propoxy]dibenzo[d,f][1,3,2]dioxaphosphepin and mixtures thereof.

Examples of the ultraviolet absorber include the following:

### (1) salicylate derivative

phenyl salicylate, 4-t-butylphenyl salicylate, 2,4-di-t-butylphenyl 3',5'-di-t-butyl-4'-hydroxybenzoate, 4-t-octylphenyl salicylate, bis(4-t-butylbenzoyl)resorcinol, benzoylresorcinol, hexadecyl 3',5'-di-t-butyl-4'-hydroxybenzoate, octadecyl 3',5'-di-t-butyl-4'-hydroxybenzoate, 2-methyl-4,6-di-t-butylphenyl 3',5'-di-t-butyl-4'-hydroxybenzoate and mixtures thereof.

### (2) 2-hydroxybenzophenone and derivative thereof

2-hydroxybenzophenone, 2,4-dihydroxybenzophenone, 2-hydroxy-4-methoxybenzophenone, 2-hydroxy-4-octoxybenzophenone, 2,2'-dihydroxy-4-methoxybenzophenone, bis(5-benzoyl-4-hydroxy-2-methoxyphenyl)methane, 2,2',4,4'-tetrahydroxybenzophenone and mixtures thereof.

### (3) 2-(2'-hydroxyphenyl)benzotriazole and derivative thereof

2-(2'-hydroxyphenyl)benzotriazole, 2-(2-hydroxy-5-methylphenyl)benzotriazole, 2-(3',5'-di-t-butyl-2'-hydroxyphenyl)benzotriazole, 2-(5'-t-butyl-2'-hydroxyphenyl)benzotriazole, 2-(2'-hydroxy-5'-t-octylphenyl)benzotriazole, 2-(3-t-butyl-2-hydroxy-5-methylphenyl)-5-chlorobenzotriazole, 2-(3'-s-butyl-2'-hydroxy-5'-t-butylphenyl)benzotriazole, 2-(2'-hydroxy-4'-octyloxyphenyl)benzotriazole, 2-(3',5'-di-t-amyl-2'-hydroxyphenyl)benzotriazole, 2-[2'-hydroxy-3',5'-bis(α,α-dimethylbenzyl)phenyl]-2H-benzotriazole, 2-[(3'-t-butyl-2'-hydroxyphenyl)-5'-(2-octyloxycarbonylethyl)phenyl]-5-chlorobenzotriazole, 2-[3'-t-butyl-5'-[2-(2-ethylhexyloxy)carbonylethyl]-2'-hydroxyphenyl]-5-chlorobenzotriazole, 2-[3'-t-butyl-2'-hydroxy-5'-(2-methoxycarbonylethyl)phenyl]-5-chlorobenzotriazole, 2-[3'-t-butyl-2'-hydroxy-5'-(2-methoxycarbonylethyl)phenyl]benzotriazole, 2-[3'-t-butyl-2'-hydroxy-5-(2-octyloxycarbonylethyl)phenyl]benzotriazole, 2-[3'-t-butyl-2'-hydroxy-5'-[2-(2-ethylhexyloxy)carbonylethyl]phenyl]benzotriazole, 2-[2-hydroxy-3-(3,4,5,6-tetrahydrophthalimidomethyl)-5-methylphenyl]benzotriazole, 2-(3,5-di-t-butyl-2-hydroxyphenyl)-5-chlorobenzotriazole, mixture of 2-(3'-dodecyl-2'-hydroxy-5'-methylphenyl)benzotriazole and 2-[3'-t-butyl-2'-hydroxy-5'-(2-isooctyloxycarbonylethyl)phenyl]benzotriazole, 2,2'-methylene bis[6-(2H-benzotriazol-2-yl)-4-(1,1,3,3-tetramethylbutyl)phenol, 2,2'-methylene bis[4-t-butyl-6-(2H-benzotriazol-2-yl)phenol], condensate of poly(3-11)(ethylene glycol) and 2-[3'-t-butyl-2'-hydroxy-5'-(2-methoxycarbonylethyl)phenyl]benzotriazole, condensate of poly(3-11)(ethylene glycol) and methyl 3-[3-(2H-benzotriazol-2-yl)-5-t-butyl-4-hydroxyphenyl]propionate, 2-ethylhexyl 3-[3-t-butyl-5-(5-chloro-2H-benzotriazol-2-yl)-4-hydroxyphenyl]propionate, octyl 3-[3-t-butyl-5-(5-chloro-2H-benzotriazol-2-yl)-4-hydroxyphenyl]propionate, methyl 3-[3-t-butyl-5-(5-chloro-2H-benzotriazol-2-yl)-4-hydroxyphenyl]propionate, 3-[3-t-butyl-5-(5-chloro-2H-benzotriazol-2-yl)-4-hydroxyphenyl]propionic acid and mixtures thereof.

Examples of the light stabilizer include the following:

### (1) hindered amine light stabilizer

bis(2,2,6,6-tetramethyl-4-piperidyl) sebacate, bis((2,2,6,6-tetramethyl-4-piperidyl) succinate, bis(1,2,2,6,6-pentamethyl-4-piperidyl) sebacate, bis(N-octoxy-2,2,6,6-tetramethyl-4-piperidyl) sebacate, bis(N-benzyloxy-2,2,6,6-tetramethyl-4-piperidyl) sebacate, bis(N-cyclohexyloxy-2,2,6,6-tetramethyl-4-piperidyl) sebacate, bis(1,2,2,6,6-pentamethyl-4-piperidyl) 2-(3,5-di-t-butyl-4-hydroxybenzyl)-2-butylmalonate, bis(1-acroyl-2,2,6,6-tetramethyl-4-piperidyl)2,2-bis(3,5-di-t-butyl-4-hydroxybenzyl)-2-butylmalonate, bis(1,2,2,6,6-pentamethyl-4-piperidyl decanedioate, 2,2,6,6-tetramethyl-4-piperidyl methacrylate, 4-[3-(3,5-di-t-butyl-4-hydroxyphenyl)propionyloxy]-1-[2-(3-(3,5-di-t-butyl-4-hydroxyphenyl)propionyloxy)ethyl]-2,2,6,6-tetramethylpiperidine, 2-methyl-2-(2,2,6,6-tetramethyl-4-piperidyl)amino-N-(2,2,6,6-tetramethyl-4-piperidyl)propionamide, tetrakis(2,2,6,6-tetramethyl-4-piperidyl) 1,2,3,4-butanetetracarboxylate, tetrakis(1,2,2,6,6-pentamethyl-4-piperidyl) 1,2,3,4-butanetetracarboxylate, mixed ester of 1,2,3,4-butanetetracarboxylic acid, 1,2,2,6,6-pentamethyl-4-piperidinol and 1-tridecanol, mixed ester of 1,2,3,4-butanetetracarboxylic acid, 2,2,6,6-tetramethyl-4-piperidinol and 1-tridecanol, mixed ester of 1,2,3,4-butanetetracarboxylic acid, 1,2,2,6,6-pentamethyl-4-piperidinol and 3,9-bis(2-hydroxy-1,1-dimethylethyl)-2,4,8,10-tetraoxaspiro[5.5]undecane, mixed ester of 1,2,3,4-butanetetracarboxylic acid, 2,2,6,6-tetramethyl-4-piperidinol and 3,9-bis(2-hydroxy-1,1-dimethylethyl)-2,4,8,10-tetraoxaspiro[5.5]undecane, polycondensate of dimethyl succinate and 1-(2-hydroxyethyl)-4-hydroxy-2,2,6,6-tetramethylpiperidine, poly[(6-morpholino-1,3,5-triazine-2,4-diyl)((2,2,6,6-tetramethyl-4-piperidyl)imino)hexamethylene((2,2,6,6-tetramethyl-4-piperidyl)imino)], poly[(6-(1,1,3,3-tetramethylbutyl)imino-1,3,5-triazine-2,4-diyl((2,2,6,6-tetramethyl-4-piperidyl)imino)hexamethylene((2,2,6,6-tetramethyl-4-piperidyl)imino)], polycondensate of N,N'-bis(2,2,6,6-tetramethyl-4-piperidyl)hexamethylenediamine and 1,2-dibromoethane, N,N',4,7-tetrakis[4,6-bis(N-butyl-N-(2,2,6,6-tetramethyl-4-piperidyl)amino)-1,3,5-triazin-2-yl]-4,7-diazadecane-1,10-diamine, N,N',4-tris[4,6-bis(N-butyl-N-(2,2,6,6-tetramethyl-4-piperidyl)amino)-1,3,5-triazin-2-yl]-4,7-diazadecane-1,10-diamine, N,N',4,7-tetrakis[4,6-bis(N-butyl-N-(1,2,2,6,6-pentamethyl-4-piperidyl)amino)-1,3,5-triazin-2-yl]-4,7-diazadecane-1,10-diamine, N,N',4-tris[4,6-bis(N-butyl-N-(1,2,2,6,6-pentamethyl-4-piperidyl)amino)-1,3,5-triazin-2-yl]-4,7-diazadecane-1,10-diamine and mixtures thereof.

### (2) acrylate light stabilizer

ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxycinnamate, methyl α-carbomethoxy-p-methoxycinnamate and N-(β-carbomethoxy-β-cyanovinyl)-2-methylindoline and mixtures thereof.

### (3) nickel light stabilizer

nickel complex of 2,2'-thiobis-[4-(1,1,3,3-tetramethylbutyl)phenol], nickel dibutyldithiocarbamate, nickel salt of monoalkylester, nickel complex of ketoxime and mixtures thereof.

### (4) oxamide light stabilizer

4,4'-dioctyloxyoxanilide, 2,2'-diethoxyoxanilide, 2,2'-dioctyloxy-5,5'-di-t-butylanilide, 2,2'-didodecyloxy-5,5'-di-t-butylanilide, 2-ethoxy-2'-ethyloxanilide, N,N'-bis(3-dimethylaminopropyl)oxamide, 2-ethoxy-5-t-butyl-2'-ethoxyanilide, 2-ethoxy-5,4'-di-t-butyl-2'-ethyloxanilide and mixtures thereof.

### (5) 2-(2-hydroxyphenyl)-1,3,5-triazine light stabilizer

2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-[2,4-dihydroxyphenyl-4,6-bis(2,4-dimethylphenyl]-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-[2-hydroxy-4-(2-hydroxy-3-butyloxypropoxy)phenyl]-4,6-bis(2,4-dimethylphenyl)-1,3,5-triazine, 2-[2-hydroxy-4-(2-hydroxy-3-octyloxypropoxy)phenyl]-4,6-bis(2,4-dimethylphenyl)-1,3,5-triazine and mixtures thereof.

Examples of the metal deactivator include the following:
N,N'-diphenyloxamide, N-salicylal-N'-salicyloylhydrazine, N,N'-bis(salicyloyl)hydrazine, N,N'-bis(3,5-di-t-butyl-4-hydroxyphenylpropionyl)hydrazine, 3-salicyloylamino-1,2,4-triazole, bis(benzylidene)oxalyl dihydrazide, oxanilide, isophthaloyl dihydrazide, sebacoyl bisphenyl hydrazide, N,N'-bis(salicyloyl)oxalyl dihydrazide, N,N'-bis(salicyloyl)thiopropionyl dihydrazide and mixtures thereof.

Examples of the peroxide scavenger include the following:
ester of β-thiodipropionic acid, mercaptobenzoimidazole, zinc salt of 2-mercaptobenzoimidazole, zinc salt of dibutyldithiocarbamic acid, dioctadecyl disulfide, pentaerythritol tetrakis(β-dodecylmercapto)propionate and mixtures thereof.

Examples of the polyamide stabilizer include copper or divalent manganese salt of iodide or phosphorous compound and mixtures thereof.

Examples of the hydroxyamine include the following:
N,N-dibenzylhydroxyamine, N,N-diethylhydroxyamine, N,N-dioctylhydroxyamine, N,N-dilaurylhydroxyamine, N,N-ditetradecylhydroxyamine, N,N-dihexadecylhydroxyamine, N,N-dioctadecylhydroxyamine, N-hexadecyl-N-octadecylhydroxyamine, N-heptadecyl-N-octadecylhydroxyamine and mixtures thereof.

Examples of the neutralizing agent include the following:
calcium stearate, zinc stearate, magnesium stearate, hydrotalcite (basic magnesium.aluminum.hydroxy.carbonate. hydrate), melamine, amine, polyamide, polyurethane and mixtures thereof.

Examples of the lubricant include the following:
aliphatic hydrocarbons such as paraffin, wax and the like, C₈₋₂₂ higher fatty acid, C₈₋₂₂ higher fatty acid metal (Al, Ca, Mg, Zn) salt, C₈₋₂₂ aliphatic alcohol, polyglycol, ester of C₄₋₂₂ higher fatty acid and C₄₋₁₈ monovalent aliphatic alcohol, C₈₋₂₂ higher aliphatic amide, silicone oil, rosin derivative.

Examples of the nucleating agent include the following:
sodium 2,2'-methylene bis(4,6-di-t-butylphenyl)phosphate, dihydrooxyaluminum [phosphate-2,2'-methylene bis(4,6-di-t-butylphenyl)],hydrooxyaluminum bis[phosphate-2,2'-methylene bis(4,6-di-t-butylphenyl)], aluminum tris[phosphate-2,2'-methylene bis(4,6-di-t-butylphenyl)], sodium bis(4-t-butylphenyl)phosphate, metal benzoate such as sodium benzoate and the like, aluminum p-t-butylbenzoate, 1,3:2,4-bis(O-benzylidene)sorbitol, 1,3:2,4-bis(O-methylbenzylidene)sorbitol, 1,3:2,4-bis(O-ethylbenzylidene)sorbitol, 1,3-O-3,4-dimethylbenzylidene-2,4-0-benzylidenesorbitol, 1,3-0-benzylidene-2,4-O-3,4-dimethylbenzylidenesorbitol, 1,3:2,4-bis(O-3,4-dimethylbenzylidene)sorbitol, 1,3-O-p-chlorobenzylidene-2,4-O-3,4-dimethylbenzylidenesorbitol, 1,3-0-3,4-dimethylbenzylidene-2,4-O-p-chlorobenzylidenesorbitol, 1,3:2,4-bis(O-p-chlorobenzylidene)sorbitol and mixtures thereof.

Examples of the filler include the following:
calcium carbonate, silicate, glass fiber, asbestos, talc, kaolin, mica, barium sulfate, carbon black, carbon fiber, zeolite and mixtures thereof.

Of the aforementioned other additives, phenolic antioxidant, phosphorus antioxidant, ultraviolet absorber, hindered amine light stabilizer, peroxide scavenger and neutralizing agent are preferable.

Particularly preferable phenolic antioxidant includes the following. Only one kind thereof may be used, or two or more kinds thereof may be used in combination.

2,6-di-t-butyl-4-methylphenol, 2,4,6-tri-t-butylphenol, 2,4-dioctylthiomethyl-6-methylphenol, 2,2'-thiobis(6-t-butylphenol), 4,4'-thiobis(3-methyl-6-t-butylphenol), 2,2'-methylene bis(4-methyl-6-t-butylphenol), 2,2'-methylene bis(4-ethyl-6-t-butylphenol), 2,2'-methylene bis[4-methyl-6-(α-methylcyclohexyl)phenol)], 2,2'-methylene bis(4-methyl-6-cyclohexylphenol), 2,2'-methylene bis(4,6-di-t-butylphenol), 2,2'-ethylidene bis(4,6-di-t-butylphenol), 4,4'-methylene bis(6-t-butyl-2-methylphenol), 4,4'-methylene bis(2,6-di-t-butylphenol), 4,4'-butylidene bis(3-methyl-6-t-butylphenol), 1,1-bis(4-hydroxyphenyl)cyclohexane, 1,1-bis(5-t-butyl-4-hydroxy-2-methylphenyl)butane, 1,1,3-tris(5-t-butyl-4-hydroxy-2-methylphenyl)butane, ethylene glycol bis[3,3-bis-3'-t-butyl-4'-hydroxyphenyl)butyrate], 2-t-butyl-6-(3'-t-butyl-5'-methyl-2'-hydroxybenzyl)-4-methylphenyl acrylate, 2,4-di-t-pentyl-6-[1-(2-hydroxy-3,5-di-t-pentylphenyl)ethyl]phenyl acrylate, 2,4,6-tris(3,5-di-t-butyl-4-phenoxy)-1,3,5-triazine, tris(4-t-butyl-3-hydroxy-2,6-dimethylbenzyl)isocyanurate, bis(3,5-di-t-butyl-4-hydroxybenzyl)isocyanurate, tris[2-(3',5'-di-t-butyl-4'-hydroxycinnamoyloxy)ethyl]isocyanurate, diethyl-3,5-di-t-butyl-4-hydroxybenzylphosphonate, di-n-octadecyl-3,5-di-t-butyl-4-hydroxybenzylphosphonate, calcium salt of 3,5-di-t-butyl-4-hydroxybenzylphosphonic acid monoester, n-octadecyl 3-(3,5-di-t-butyl-4-hydroxyphenyl) propionate, neopentanetetrayl tetrakis(3,5-di-t-butyl-4-hydroxycinnamate), thiodiethylene bis(3,5-di-t-butyl-4-hydroxycinnamate), 1,3,5-trimethyl-2,4,6-tris(3,5-di-t-butyl-4-hydroxybenzyl)benzene, 3,6-dioxaoctamethylene bis(3,5-di-t-butyl-4-hydroxycinnamate), hexamethylene bis(3,5-di-t-butyl-4-hydroxycinnamate), triethylene glycol bis(5-t-tyl-4-hydroxy-3-methylcinnamate), 3,9-bis[2-(3-(3-t-butyl-4-hydroxy-5-methylphenyl)propionyloxy)-1,1-dimethylethyl]-2,4,8,10-tetraoxaspiro[5.5]undecane, N,N'-bis[3-(3',5'-di-t-butyl-4'-hydroxyphenyl)propionyl]hydrazine, N,N'-bis[3-(3',5'-di-t-butyl-4'-hydroxyphenyl)propionyl]hexamethylenediamine.

Particularly preferable phosphorus antioxidant includes the following. Only one kind thereof may be used, or two or more kinds thereof may be used in combination.

tris(nonylphenyl) phosphite, tris(2,4-di-t-butylphenyl) phosphite, distearyl pentaerythritol diphosphite, bis(2,4-di-t-butylphenyl) pentaerythritol diphosphite, bis(2,4-di-t-butyl-6-methylphenyl) pentaerythritol diphosphite, bis(2,6-di-t-butyl-4-methylphenyl) pentaerythritol diphosphite, tetrakis(2,4-di-t-butylphenyl)-4,4'-diphenylene diphosphonite, 2,2'-methylene bis(4,6-di-t-butylphenyl) 2-ethylhexyl phosphite, 2,2'-ethylidene bis(4,6-di-t-butylphenyl) fluoro phosphite, bis(2,4-di-t-butyl-6-methylphenyl) ethyl phosphite, 2-(2,4,6-tri-t-butylphenyl)-5-ethyl-5-butyl-1,3,2-oxaphosphorinan, 2,2',2"-nitrilo[triethyl-tris(3,3',5,5'-tetra-t-butyl-1,1'-biphenyl-2,2'-diyl) phosphite, 2,4,8,10-tetra-t-butyl-6-[3-(3-methyl-4-hydroxy-5-t-butylphenyl)propoxy]dibenzo[d,f][1,3,2]dioxaphosphepin.

Particularly preferable ultraviolet absorber includes the following. Only one kind thereof may be used, or two or more kinds thereof may be used in combination.

phenyl salicylate, 4-t-butylphenyl salicylate, 2,4-di-t-butylphenyl 3',5'-di-t-butyl-4'-hydroxybenzoate, 4-t-octylphenyl salicylate, 2,4-dihydroxybenzophenone, 2-hydroxy-4-methoxybenzophenone, 2-hydroxy-4-octoxybenzophenone, 2,2'-dihydroxy-4-methoxybenzophenone, bis(5-benzoyl-4-hydroxy-2-methoxyphenyl)methane, 2,2',4,4'-tetrahydroxybenzophenone, 2-(2-hydroxy-5-methylphenyl)benzotriazole, 2-(3',5'-di-t-butyl-2'-hydroxyphenyl)benzotriazole, 2-(5'-t-butyl-2'-hydroxyphenyl)benzotriazole, 2-(2'-hydroxy-5'-t-octylphenyl)benzotriazole, 2-(3-t-butyl-2-hydroxy-5-methylphenyl)-5-chlorobenzotriazole, 2-(3'-s-butyl-2'-hydroxy-5'-t-butylphenyl)benzotriazole, 2-(2'-hydroxy-4'-octyloxyphenyl)benzotriazole, 2-(3',5'-di-t-amyl-2'-hydroxyphenyl)benzotriazole, 2-[2'-hydroxy-3',5'-bis(α,α-dimethylbenzyl)phenyl]-2H-benzotriazole.

Particularly preferable light stabilizer includes the following. Only one kind thereof may be used, or two or more kinds thereof may be used in combination.

bis(2,2,6,6-tetramethyl-4-piperidyl) sebacate, bis(1,2,2,6,6-pentamethyl-4-piperidyl) sebacate, bis(N-octoxy-2,2,6,6-tetramethyl-4-piperidyl) sebacate, bis(N-benzyloxy-2,2,6,6-tetramethyl-4-piperidyl) sebacate, bis(N-cyclohexyloxy-2,2,6,6-tetramethyl-4-piperidyl) sebacate, bis(1,2,2,6,6-pentamethyl-4-piperidyl) 2-(3,5-di-t-butyl-4-hydroxybenzyl)-2-butylmalonate, bis(1-acroyl-2,2,6,6-tetramethyl-4-piperidyl) 2,2-bis(3,5-di-t-butyl-4-hydroxybenzyl)-2-butylmalonate, bis(2,2,6,6-tetramethyl-4-piperidyl) succinate, 2,2,6,6-tetramethyl-4-piperidyl methacrylate, 4-[3-(3,5-di-t-butyl-4-hydroxyphenyl)propionyloxy]-1-[2-(3-(3,5-di-t-butyl-4-hydroxyphenyl)propionyloxy)ethyl]-2,2,6,6-tetramethylpiperidine, 2-methyl-2-(2,2,6,6-tetramethyl-4-piperidyl)amino-N-(2,2,6,6-tetramethyl-4-piperidyl)propionamide, tetrakis(2,2,6,6-tetramethyl-4-piperidyl) 1,2,3,4-butanetetracarboxylate, tetrakis(1,2,6,6-pentamethyl-4-piperidyl) 1,2,3,4-butanetetracarboxylate, mixed ester of 1,2,3,4-butanetetracarboxylic acid, 1,2,2,6,6-pentamethyl-4-piperidinol and 1-tridecanol, mixed ester of 1,2,3,4-butanetetracarboxylic acid, 2,2,6,6-tetramethyl-4-piperidinol and 1-tridecanol, mixed ester of 1,2,3,4-butanetetracarboxylic acid, 1,2,2,6,6-pentamethyl-4-piperidinol and 3,9-bis(2-hydroxy-1,1-dimethylethyl)-2,4,8,10-tetraoxaspiro[5.5]undecane, mixed ester of 1,2,3,4-butanetetracarboxylic acid, 2,2,6,6-tetramethyl-4-piperidinol and 3,9-bis(2-hydroxy-1,1-dimethylethyl)-2,4,8,10-tetraoxaspiro[5.5]undecane, polycondensate of dimethyl succinate and 1-(2-hydroxyethyl)-4-hydroxy-2,2,6,6-tetramethylpiperidine, poly[(6-morpholino-1,3,5-triazine-2,4-diyl)((2,2,6,6-tetramethyl-4-piperidyl)imino)hexamethylene((2,2,6,6-tetramethyl-4-piperidyl)imino)], poly[(6-(1,1,3,3-tetramethylbutyl)-1,3,5-triazine-2,4-diyl) ((2,2,6,6-tetramethyl-4-piperidyl)imino)hexamethylene((2,2,6,6-tetramethyl-4-piperidyl)imino)].

In the production of a stabilizer containing other additives, a method of mixing compound (I) and other additive is not particularly limited, and a known method and an apparatus can be used. In the production of an organic material composition, a method of mixing compound (I), an organic material and other additive as necessary is not particularly limited, and a known method and an apparatus can be used.

When the organic material is a solid polymer, (1) compound (I) may be directly dry blended with the solid polymer, (2) a small amount of a solid polymer and compound (I) are first kneaded to form a master batch, and then the master batch and a solid polymer may be dry blended. When the organic material is a liquid polymer, a solution or dispersion of compound (I) may be added to a polymer solution during polymerization or after polymerization. When the organic material is a liquid such as lubricating oil and the like, (1) compound (I) may be directly added to a liquid organic material, (2) compound (I) is dissolved or dispersed in a solvent to prepare a solution or dispersion, and the obtained solution or dispersion may be added to the liquid organic material.

### [Examples]

The present invention is explained in more detail in the following by referring to Examples, which do not limit the present invention. It is also possible to carry out the present invention by making appropriate modifications within the range that can conform to the above and the following gist, all of which are encompassed in the technical scope of the present invention

The "part" described in the following Examples and the like means "part by weight".

### Production Example 1: Production of 2,4-di-t-butyl-6-(2,4-di-t-butylphenoxy)phenol

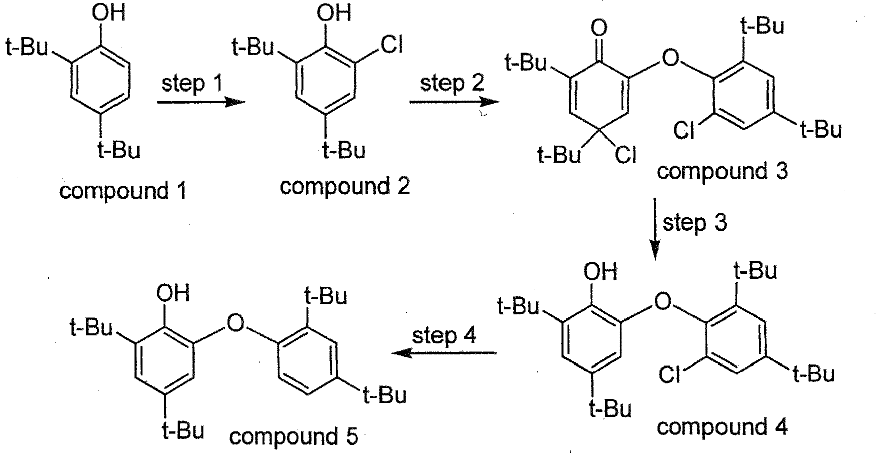

### (Step 1)

Compound 1 (2,4-di-t-butylphenol) (25.0 g), SO₂Cl₂ (16.38 g) and diethyl ether (250 mL) were charged in a four-necked flask, and stirred under a nitrogen atmosphere at 25°C for 4 hr. The solvent in the obtained reaction solution was evaporated under reduced pressure at not more than 40°C, and the concentrated residue was purified by silica gel column chromatography (solvent: ethyl acetate, hexane). The solvent in the purified solution was evaporated under reduced pressure at not more than 40°C to give compound 2 (24.1 g).

### (Step 2)

Compound 2 (35.0 g) produced in the same manner as in step 1, K₃Fe(CN)₆ (239.3 g) and KOH (239.3 g) were dissolved in water (3.5 L), benzene (2.4 L) was added to the obtained solution, and the reaction solution was stirred under a nitrogen atmosphere at 25°C for 20 min. Using a mixed solvent of CCl₄:n-hexane=1:9 (volume ratio), compound 3 was extracted from the reaction solution, and the solvent in the extracted solution was evaporated under reduced pressure at not more than 40°C to give compound 3 (23.2 g).

### (Step 3)

Compound 3 (23.0 g) and LiAlH₄ (5.46 g) were dissolved in THF (460 mL), and the reaction solution was stirred at 0°C for 20 min. The obtained reaction solution was filtered to remove the residue. The solvent in the filtrate was evaporated under reduced pressure at not more than 40°C, and the concentrated residue was purified by silica gel column chromatography (solvent: ethyl acetate, hexane). The solvent in the purified solution was evaporated under reduced pressure at not more than 40°C to give compound 4 (19.5 g).

### (Step 4)

Under a nitrogen atmosphere at 25°C, compound 4 (9 g), t-BuOK (9.08 g), and diethyl ether (180 mL) were mixed. Thereafter, the system was maintained at -10°C, and a 2.5 M n-BuLi hexane solution (32.3 mL) was added dropwise over not less than 20 min. Then, the mixture was stirred at -10°C for 2 hr. The reaction was quench with water (3.6 mL), and the reaction solution was filtered to remove the residue. The solvent in the filtrate was evaporated under reduced pressure at not more than 40°C, and the concentrated residue was purified by silica gel column chromatography (solvent: ethyl acetate, hexane). The solvent in the purified solution was evaporated under reduced pressure at not more than 40°C to give compound 5 (2,4-di-t-butyl-6-(2,4-di-t-butylphenoxy)phenol, 5.8 g). Obtainment of compound 5 was confirmed by ¹H-NMR measurement.
¹H-NMR
apparatus: "AV-600" manufactured by Bruker
resonance frequency: 600 MHz
measurement solvent: CDCl₃
internal standard: tetramethylsilane
δ:7.42(d, J=3.60Hz, 1H, Ar-H), 7.11, 7.07(m, 2H, Ar-H), 6.75(d, J=3.20Hz, 1H, Ar-H), 6.65(d, J=13.2Hz, 1H, Ar-H), 5.72(s, 1H, OH), 1.49, 1.45, 1.32, 1.23(s, 9H, C-(CH₃)₃)

### Example 1

To a powder (100 parts) of linear low-density polyethylene ("FS150" manufactured by Sumitomo Chemical Company, Limited, melt flow rate (MFR) at temperature 190°C and load 21.18N: 1.0 g/10 min) were added 2,4-di-t-butyl-6-(2,4-di-t-butylphenoxy)phenol (0.05 part) (0.0012 mol per 1 kg of polyethylene) and calcium stearate (0.05 part), and the mixture was dry blended. Then, the obtained blend was granulated using a 30 mmϕ single screw extruder at 190°C to give pellets. The oxidation induction time of the obtained pellet was measured by DSC apparatus ("DSC7020" manufactured by Hitachi High-Tech Science Corporation). To be specific, under a nitrogen atmosphere, the temperature of the pellets was raised at 20°C/min, and when the pellets reached 200°C, the atmosphere was changed to oxygen atmosphere at an oxygen flow rate of 100 mL/min, and the time period from the change into oxygen atmosphere to the appearance of the exothermic peak was measured as an oxidation induction time (i.e., oxidation induction time = when exothermic peak appeared - when changed into oxygen atmosphere) . In addition, the time when the exothermic peak appeared after change into oxygen atmosphere was determined as a time point for an intersection point of the tangent line of a DSC curve when an exothermic amount is 0 mW (i.e., approximately parallel to horizontal axis), and the tangent line of an inflection point of the DSC curve. The results are shown in Table 1. A longer pellet (composition) oxidation induction time means higher oxidation resistance.

### Comparative Example 1

In the same manner as in Example 1 except that 2,4-di-t-butyl-6-(2,4-di-t-butylphenoxy)phenol was not added, oxidation induction time was measured. The results are shown in Table 1.

**Table 1**

| | oxidation induction time (min) |
|---|---|
| Example 1 | 10.14 |
| Comparative Example 1 | 0.56 |

As shown in Table 1,2,4-di-t-butyl-6-(2,4-di-t-butylphenoxy)phenol can improve oxidation resistance of pellets (composition) containing polyethylene.

### Example 2

To a powder (100 parts) of polypropylene ("AW630G" manufactured by Sumitomo Chemical Company, Limited, melt flow rate (MFR) at temperature 230°C and load 21.18N: 10.0 g/10 min) were added 2,4-di-t-butyl-6-(2,4-di-t-butylphenoxy)phenol (0.05 part) (0.0012 mol per 1 kg of polypropylene) and calcium stearate (0.05 part), and the mixture was dry blended. Then, the obtained blend was granulated using a 30 mmϕ single screw extruder at 230°C to give pellets. The melt flow rate (MFR, g/10 min) of the obtained pellet was measured by "melt indexer L246-3537" manufactured by Technol Seven Co., Ltd. under the conditions of temperature in cylinder 230°C, dwell time 5 min, load 21.18N (2.16 kg). The results are shown in Table 2.

Polypropylene is known to be thermally decomposed during extrusion molding and degraded. When polypropylene is degraded, MFR of the pellets (composition) containing same increases. Therefore, a low MFR of the pellets containing polypropylene means that the thermal decomposition of polypropylene is suppressed and the processing stability thereof is high.

### Comparative Example 2

In the same manner as in Example 2 except that 2,4-di-t-butyl-6-(2,4-di-t-butylphenoxy)phenol was not added, MFR was measured. The results are shown in Table 2.

### Comparative Example 3

In the same manner as in Example 2 except that "IRGANOX (registered trade mark) 1076" (octadecyl 3-(3,5-di-t-butyl-4-hydroxyphenyl)propionate) (0.065 part) (0.0012 mol per 1 kg of polypropylene) manufactured by BASF was added instead of 2,4-di-t-butyl-6-(2,4-di-t-butylphenoxy)phenol (0.05 part), MFR was measured. The results are shown in Table 2.

**Table 2**

| | MFR (g/10 min) |
|---|---|
| Example 2 | 16.0 |
| Comparative Example 2 | 19.4 |
| Comparative Example 3 | 17.2 |

As shown in Table 2, the MFR of the pellets of Example 2 containing 2,4-di-t-butyl-6-(2,4-di-t-butylphenoxy)phenol is lower than that of the pellets of Comparative Example 2 free of a stabilizer and that of the pellets of Comparative Example 3 containing conventional IRGANOX (registered trade mark) 1076, and 2,4-di-t-butyl-6-(2,4-di-t-butylphenoxy)phenol is superior for the improvement of the processing stability of pellets (composition) containing polypropylene.

### [Industrial Applicability]

The compound (I) of the present invention can improve processing stability and oxidation resistance of an organic material composition, and is useful as a stabilizer.

## Claims

1. A compound represented by the formula (I): 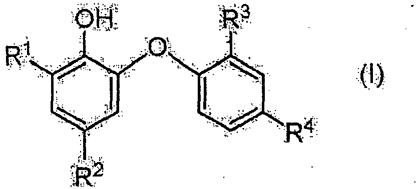 in the formula (I), R¹ - R⁴ are each independently a t-butyl group or a t-pentyl group.

2. The compound according to claim 1, wherein R¹ - R⁴ are each a t-butyl group.

3. A stabilizer for an organic material, comprising the compound according to claim 1 or 2.

4. The stabilizer according to claim 3, wherein the organic material is a thermoplastic resin.

5. The stabilizer according to claim 4, wherein the thermoplastic resin is a polyolefin, a polyamide,
a polyester, or a polycarbonate.

6. A method of stabilizing an organic material, comprising mixing the organic material and the compound according to claim 1 or 2.

7. The method according to claim 6, wherein the organic material is a thermoplastic resin.

8. The method according to claim 7, wherein the thermoplastic resin is a polyolefin, a polyamide,
a polyester, or a polycarbonate.

9. A stabilized organic material composition comprising an organic material and the compound according to claim 1 or 2.

10. The organic material composition according to claim 9, wherein the organic material is a thermoplastic resin.

11. The organic material composition according to claim 10, wherein the thermoplastic resin is a polyolefin, a polyamide, a polyester, or a polycarbonate.

## Patentansprüche

1. Eine Verbindung, die durch die Formel (I) dargestellt ist: 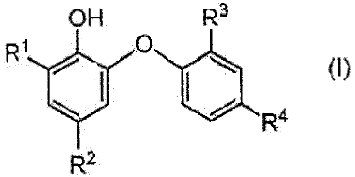 wobei in der Formel (I) R¹-R⁴ jeweils unabhängig eine t-Butylgruppe oder eine t-Pentylgruppe sind.

2. Die Verbindung nach Anspruch 1, wobei R¹-R⁴ jeweils eine t-Butylgruppe sind.

3. Ein Stabilisator für ein organisches Material, umfassend die Verbindung nach Anspruch 1 oder 2.

4. Der Stabilisator nach Anspruch 3, wobei das organische Material ein thermoplastisches Harz ist.

5. Der Stabilisator nach Anspruch 4, wobei das thermoplastische Harz ein Polyolefin, ein Polyamid, ein Polyester oder ein Polycarbonat ist.

6. Ein Verfahren zum Stabilisieren eines organischen Materials, umfassend Mischen des organischen Materials und der Verbindung nach Anspruch 1 oder 2.

7. Das Verfahren nach Anspruch 6, wobei das organische Material ein thermoplastisches Harz ist.

8. Das Verfahren nach Anspruch 7, wobei das thermoplastische Harz ein Polyolefin, ein Polyamid, ein Polyester oder ein Polycarbonat ist.

9. Eine stabilisierte organische Materialzusammensetzung, umfassend ein organisches Material und die Verbindung nach Anspruch 1 oder 2.

10. Die organische Materialzusammensetzung nach Anspruch 9, wobei das organische Material ein thermoplastisches Harz ist.

11. Die organische Materialzusammensetzung nach Anspruch 10, wobei das thermoplastische Harz ein Polyolefin, ein Polyamid, ein Polyester oder ein Polycarbonat ist.

## Revendications

1. Composé représenté par la formule (I): 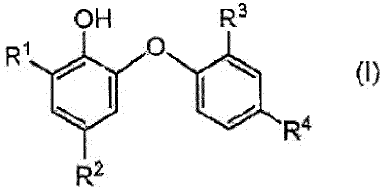 dans la formule (I), R¹-R⁴ sont chacun indépendamment un groupe t-butyle ou un groupe t-pentyle.

2. Composé selon la revendication 1, R¹-R⁴ étant chacun un groupe t-butyle.

3. Stabilisant pour matériau organique, comprenant le composé selon la revendication 1 ou 2.

4. Stabilisant selon la revendication 3, le matériau organique étant une résine thermoplastique.

5. Stabilisant selon la revendication 4, la résine thermoplastique étant une polyoléfine, un polyamide, un polyester, ou un polycarbonate.

6. Procédé de stabilisation d'un matériau organique, comprenant le mélange du matériau organique et du composé selon la revendication 1 ou 2.

7. Procédé selon la revendication 6, le matériau organique étant une résine thermoplastique.

8. Procédé selon la revendication 7, la résine thermoplastique étant une polyoléfine, un polyamide, un polyester, ou un polycarbonate.

9. Composition de matériau organique stabilisée comprenant un matériau organique et le composé selon la revendication 1 ou 2.

10. Composition de matériau organique selon la revendication 9, le matériau organique étant une résine thermoplastique.

11. Composition de matériau organique selon la revendication 10, la résine thermoplastique étant une polyoléfine, un polyamide, un polyester, ou un polycarbonate.
